Europäisches Patentamt

European Patent Office

Office européen des brevets

(19)

(11) **EP 1 219 315 A1**

(12) **DEMANDE DE BREVET EUROPEEN**

(43) Date de publication:
**03.07.2002 Bulletin 2002/27**

(51) Int Cl.⁷: **A61M 16/00**

(21) Numéro de dépôt: **00811237.7**

(22) Date de dépôt: **22.12.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(71) Demandeur: **Rochat, Jean-Denis**
**1272 Genolier (CH)**

(72) Inventeur: **Rochat, Jean-Denis**
**1272 Genolier (CH)**

(74) Mandataire: **Savoye, Jean-Paul et al**
**Moinas & Savoye S.A.,**
**42, rue Plantamour**
**1201 Genève (CH)**

(54) **Appareil d'assistance respiratoire**

(57) Selon ce procédé de régulation en boucle ouverte d'un appareil d'assistance respiratoire, on forme une enceinte de compression (1), en la divisant en deux compartiments (3, 4) reliés chacun à une admission (5, 7) d'air ou de gaz respiratoire et à une évacuation (6, 8) d'air comprimé, à l'aide d'une membrane flottante élastique (2), on guide cette membrane flottante (2) en fixant sa périphérie à la paroi de ladite enceinte (1), on fixe une bobine motrice (14) au centre de ladite membrane flottante (2), on place cette bobine motrice (14) dans un entrefer (E) que l'on oriente dans le sens de déformation de ladite membrane flottante (2), on mesure le débit instantané d'air sortant par ladite évacuation (6, 8) et on alimente ladite bobine motrice (14) en calculant en continu l'intensité instantanée et le sens du courant d'alimentation en fonction de la pression de consigne de l'air comprimé, dudit débit instantané et des constantes dudit appareil.

Fig. 1

## EP 1 219 315 A1

**Description**

La présente invention se rapporte à un procédé de régulation de pression en boucle ouverte d'un appareil d'assistance respiratoire, ainsi qu'à un dispositif de compression d'un appareil de compression pour la mise en oeuvre de ce procédé.

Le problème rencontré avec les appareils d'assistance respiratoires qui sont appelés à fournir un débit d'air variable à pression constante est celui du temps de réponse. Il est en effet nécessaire d'arriver à produire une pression de consigne endotrachéale ajustable par le médecin, qui soit indépendante du débit d'inspiration instantané demandé par le patient, l'expiration passant par une valve expiratoire, la valve inspiratoire étant alors fermée.

Deux types d'appareils d'assistance respiratoire existent. Les appareils du premier type comportent une alimentation en gaz respiratoire sous pression dont le débit et la pression sont régulés par une valve de réglage à étranglement variable. Les appareils du second type ne comportent pas d'alimentation en gaz sous pression, mais un compresseur à pression et débits variables.

Les appareil existants fonctionnent avec un feed-back de pression, ce qui oblige à un compromis entre stabilité du système en boucle fermée et son temps de réponse. Le temps de réponse de tels systèmes est de l'ordre de 50 à 150 ms, avec le temps de réponse de la valve qui est de l'ordre de 4 à 10 ms.

Il ne serait pas possible de travailler en boucle ouverte avec un tel système, compte tenu des frottements qui ne constituent pas un paramètre fixe et dont la mesure serait trop complexe. Pour travailler en boucle ouverte, il est donc tout d'abord indispensable de trouver un dispositif de compression travaillant pratiquement sans frottement mécanique.

On a déjà proposé dans le FR-2733688 un appareil d'assistance respiratoire dont la source de gaz sous pression est un compresseur à membrane à actionnement électromagnétique. Ce compresseur comporte un carter qui renferme deux enceintes à volumes variables comprenant un arbre de guidage qui traverse un noyau de fer doux disposé coaxialement au centre d'un aimant annulaire et qui porte à chacune de ses extrémités une plaque circulaire rigide munie d'une membrane annulaire dont la périphérie est fixée à la paroi interne du carter, délimitant ainsi à l'intérieur du carter deux enceintes dont les volumes respectifs varient en fonction des déplacement des plaques et des membranes. Chaque enceinte comporte au moins un clapet d'entrée et au moins un clapet de sortie pour contrôler l'entrée et la sortie de l'air.

Un tel dispositif de compression ne répond pas aux exigences d'un système fonctionnant en boucle ouverte, étant donné que l'arbre de guidage constitue une source importante de frottement.

Le but de la présente invention est de permettre d'apporter une solution à la régulation d'un respirateur comprenant un compresseur à membrane, de manière que son temps de réponse soit réduit pratiquement à celui de la membrane du dispositif de compression.

A cet effet, la présente invention a tout d'abord pour objet un procédé de régulation en boucle ouverte d'un appareil d'assistance respiratoire, selon la revendication 1. Elle a également pour objet un appareil d'assistance respiratoire selon la revendication 3.

L'avantage de ce procédé et de l'appareil pour sa mise en oeuvre vient du fait qu'en l'absence de frottement mécanique, il suffit de mesurer le débit et de connaître la pression de consigne pour calculer le courant d'alimentation instantané de la bobine motrice, les autres paramètres étant constitués par des constantes de l'appareil d'assistance respiratoire.

Le dessin annexé illustre, schématiquement et à titre d'exemple, une forme d'exécution de l'appareil d'assistance respiratoire pour la mise en oeuvre du procédé objet de la présente invention.

La figure 1 est une vue en coupe axiale du dispositif de compression objet de la présente invention ;
la figure 2 est une vue selon II-II de la figure 1 ;
la figure 3 est un schéma-bloc du système de régulation du respirateur.

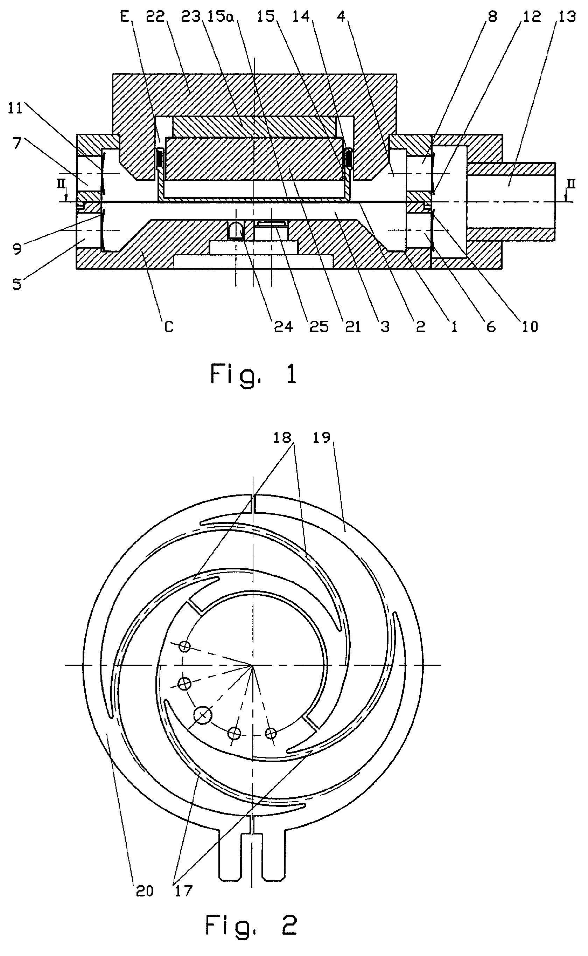

Pour permettre la mise en oeuvre du procédé de régulation selon l'invention, on réalise un dispositif de compression tel qu'illustré par la figure 1 et qui comporte un carter C à l'intérieur duquel est ménagée une enceinte de compression 1 divisée en deux compartiments 3 et 4 par une membrane élastique flottante 2 en silicone dont la périphérie est fixée à la paroi de cette enceinte 1. Cette membrane flottante 2 assure l'étanchéité entre les deux compartiments. Cette membrane 2 est dite flottante parce qu'elle est entièrement guidée par la fixation de sa périphérie à la paroi de l'enceinte 1 à l'exclusion de tout autre guidage engendrant des frottements mécaniques constituant un paramètre important et essentiellement variable qui ne permet pas la régulation en boucle ouverte.

De préférence, cette membrane flottante 2 est soumise à une certaine pré-tension lors de sa fixation à la paroi de l'enceinte 1. A cet effet, on allonge diamétralement la membrane de 3 à 8%, de préférence 4 à 5%. Cette pré-tension a pour but de limiter le battement mort de la membrane lors de l'inversion de la pression qui s'exerce sur elle.

Chaque compartiment 3, 4 communique avec l'extérieur par deux ouvertures 5, 6, respectivement 7, 8. De préférence, chaque compartiment comporte une pluralité de clapets d'entrée et de sortie. Chacune de ces ouvertures 5-8 est contrôlée par un clapet anti-retour, respectivement 9 à 12. Les deux clapets 9, 10, respectivement 11, 12 de chaque compartiment 3, 4 fonctionnent de manière inversée l'un par rapport à l'autre, de sorte que les clapets 9 et 11 permettent à l'air d'entrer dans les compartiments respectifs 3, 4, mais l'empêche de ressortir, alors que les clapets 10 et 12 permettent à l'air de sortir de ces mêmes compartiments 3, 4, mais pas d'entrer.

Les ouvertures d'admission 5, 7 communiquent avec l'atmosphère, tandis que les ouvertures 6 et 8 débouchent dans un conduit de sortie 13 destiné à conduire l'air sous pression dans le conduit trachéal du patient.

La membrane flottante 2, de préférence de forme circulaire, porte une bobine motrice 14 de forme cylindrique. Cette bobine motrice 14 est enroulée sur un cylindre creux 15 en plastique, (figures 1) dont le fond 15a est solidaire de la membrane flottante 2. Deux bras élastiques arqués 17, 18 en alliage Cu-Be, relient respectivement deux demis disques en cuivre 16a, 16b, disposés entre le fond 15a du cylindre creux 15 et la membrane flottante 2, à deux demis anneaux 19, 20. En variante, chacun de ces bras arqués 17, 18 pourrait aussi être divisé en plusieurs bras parallèles. Ces deux bras élastiques 17, 18 sont symétriques par rapport au centre de la membrane flottante 2. Les deux demis anneaux 19, 20 sont fixés entre deux parties du carter C et sont connectés aux deux pôles respectifs d'une source de courant I d'alimentation de la bobine motrice 14.

Ces deux bras 17, 18 servent également à centrer l'équipage mobile formé par la membrane flottante 2 et la bobine motrice 14 et à guider cet équipage au cours de son déplacement.

Cette bobine motrice 14 est disposée dans un entrefer E ménagé entre un noyau de fer doux 21 et une culasse en fer doux 22 qui sont reliés respectivement aux deux pôles d'un aimant permanent 23 et formant un moteur électrodynamique où la force magnétique est essentiellement indépendante de la position de la bobine.

Le dispositif de compression comporte encore une diode électroluminescente 24 disposée vis-à-vis d'une surface réfléchissante portée par la membrane flottante 2 et une photodiode 25 destinée à capter la lumière réfléchie par la surface réfléchissante, en fonction de la position de la membrane flottante 2, dont le déplacement est induit par la bobine motrice 14 en fonction de l'intensité du courant I qu'elle reçoit.

Comme on peut le constater de la description du dispositif de compression décrit ci-dessus, ce dispositif dont l'élément mobile est constitué par la membrane flottante 2 ne comporte aucune autre pièce mobile génératrice de frottement mécanique, les seuls frottements étant ceux qui se produisent dans la matière de la membrane flottante 2 et des bras élastiques 17, 18, mais ces frottements entrent dans les constantes du dispositif et sont de très faible valeur.

En plus, le dispositif de compression ne comportant qu'une seule membrane flottante, la masse en mouvement est réduite au minimum de même que l'émission de bruit. La membrane flottante 2 nécessite une bobine motrice 14 de faible hauteur dans l'entrefer E, pour que la répartition de la force exercée sur la membrane flottante 2 soit uniforme quelle que soit la position de cette membrane flottante 2. Il faut que $h_{entrefer} \geq course_{bobine} + h_{bobine}$.

Une bobine 14 de faible hauteur a aussi l'avantage de diminuer sa résistance, donc les pertes par effet Joule ($RI^2$), améliorant ainsi le rendement global du dispositif de compression.

Grâce au fait qu'avec un tel dispositif de compression, la seule variable, pour une pression de consigne donnée de l'air sous pression fourni au conduit de sortie 13, est le débit demandé qui est fonction de l'aspiration instantanée, donc de la dépression engendrée par le patient, il devient tout à fait possible de réguler l'appareil d'assistance respiratoire par un système de boucle ouverte, ce qui permet de réduire entre 5 et 30 fois le temps de réponse par rapport à un système de régulation avec feed-back.

La mesure de débit peut se faire avantageusement en détectant la position de la membrane flottante 2, dont la surface peut être réfléchissante ou peut être associée à un élément réfléchissant, à l'aide de la diode électroluminescente 24 qui envoie un spot lumineux avec un certain angle d'incidence, par exemple 60° sur la surface réfléchissante, la photodiode 25 recevant la lumière réfléchie par la membrane flottante 2, en fonction de la position de cette membrane flottante 2. De préférence, l'amplitude de la membrane flottante 2 est fixe.

La pression de l'air à la sortie du dispositif de compression doit être :

$$P_{aw} = \Delta P_{ETT} + P_e$$

$$= R \cdot \dot{V}(t) + R_2 \cdot \dot{V}^2(t) + P_e$$

où :

$P_{aw}$ = pression de sortie d'air du dispositif de compression
$P_e$ = pression endotrachéale de consigne

$P_{ETT}$ = perte de charge dans la tubulure de l'appareil d'assistance respiratoire, principalement de la canule d'intubation

$$V(t) = y(t) \cdot S$$

$S$ = surface efficace de la membrane

d'où :

$$\dot{V}(t) = \dot{y}(t) \cdot S$$

La loi de Newton appliquée à l'équipage mobile, soit la bobine motrice 14, la membrane flottante 2, les bras élastiques 17, 18 est :

$$\Sigma F = m \cdot a = m \cdot \ddot{y}(t)$$

$$P_{aw}(t) \cdot S + BlI(t) + k \cdot y(t) + \eta \cdot \dot{y}(t) = m\ddot{y}(t)$$

en introduisant

$$P_{aw} = R\dot{V}(t) + R_2 \dot{V}^2 + P_e$$

où :

$\eta$ = frottement interne de la membrane flottante
$k$ = constante de ressort du système

on obtient :

$$I(t) = -\frac{1}{Bl}[SR\dot{V}(t) + SR_2 \dot{V}^2(t) + SP_e + k \cdot y(t) + \eta \cdot \dot{y}(t) - m \cdot \ddot{y}(t)]$$

équation dans laquelle :

$$y(t) = \frac{V(t)}{S}$$

$$\dot{y} = \frac{\dot{V}}{S}$$

$$\ddot{y} = \frac{\ddot{V}}{S}$$

Soit :

$$I(t) = -\frac{1}{Bl}\left[\left(SR + \frac{\eta}{S}\right)\dot{V}(t) + SR_2\dot{V}^2(t) + \frac{k}{S}V(t) - m\frac{\ddot{V}(t)}{S} + SP_e\right]$$

A part : $I(P_e) = \frac{S \cdot P_e}{Bl}$, les autres termes de l'équation sont des corrections fonctions du débit, permettant de garder $P_e$ constant quel que soit $t$ et $\dot{V}(t)$.

Le courant dans la bobine motrice 14 peut être calculé en continu soit par un calculateur analogique soit, avantageusement à l'aide d'un processeur de traitement de signal numérique (DSP) ou d'un micro-contrôleur par lequel :

| | |
|---|---|
| **B,l,S,**$\eta$**,k,m** | sont des constantes de construction connues du respirateur. |
| R,R$_2$ | sont des constantes dépendant de la canule introduite dans la trachée du patient. Ces constantes sont entrées dans le système de régulation à l'aide d'un clavier et sont déterminées par étalonnage préliminaire avant ventilation du patient. |
| $V(t), \dot{V}(t), \ddot{V}(t)$ | sont calculés sur la base de $V(t)=y(t)\cdot S$ où $y(t)$ est mesuré par la photodiode 25. |
| $P_e$ | est la consigne de pression endotrachéale désirée et introduite dans le système de régulation à l'aide d'un clavier par le médecin. |

Le débit instantané et la fréquence instantanée du dispositif de compression décrit sont variables en fonction de la demande du patient.

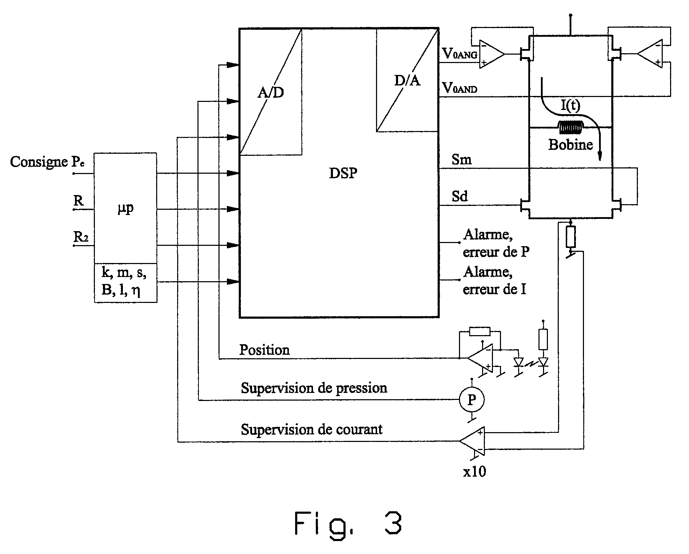

Comme illustré par la figure 3, le système de régulation pour la mise en oeuvre du procédé objet de l'invention comporte de préférence un processeur de traitement de signal numérique DSP dont les entrées sont connectées de manière à recevoir les différentes valeurs entrant dans le calcul de l'intensité du courant d'alimentation de la bobine motrice 14. Les valeurs introduites dans le DSP comportent d'une part des valeurs numériques, à savoir, les constantes **k,**$\eta$**,m,S,B,l,** les valeurs R, R$_e$, P$_e$, d'autre part des valeurs analogiques, celles fournies par la photodiode 25, celle de la mesure du courant d'alimentation en fonction du temps I$_{mes}$(t) et celle d'un capteur de pression de sécurité P$_s$, dont le rôle est de déclencher une alarme si la pression mesurée s'écartait de la valeur de consigne.

Ces valeurs analogiques sont introduites dans un convertisseur analogique digital A/D du processeur DSP et le courant d'alimentation de la bobine motrice 14 est généré par un amplificateur bi-directionnel de puissance Amp alimenté par une tension de 12 ou de 24 V, dont les entrées sont reliées à la sortie d'un convertisseur digital analogique D/A du processeur DSP, pour moduler l'intensité du courant d'alimentation en fonction de la valeur calculée par le processeur DSP. Celui-ci présente encore deux sorties S$_m$, S$_d$ qui déterminent le sens de passage du courant dans la bobine motrice 14 et donc le sens de déplacement de cette bobine 14 dans l'entrefer entre le noyau de fer doux 21 et la culasse de fer doux 22 en fonction de sa position, déterminant ainsi l'amplitude de son déplacement dans cet entrefer et jouant en quelque sorte le rôle de « butées électronique » entre lesquelles la membrane flottante 2 se déplace.

**Revendications**

1. Procédé de régulation de pression en boucle ouverte d'un appareil d'assistance respiratoire, **caractérisé en ce que** l'on forme une enceinte de compression (1), en la divisant en deux compartiments (3, 4) reliés chacun à une admission (5, 7) d'air ou de gaz respiratoire et à une évacuation (6, 8) d'air comprimé, à l'aide d'une membrane flottante élastique (2), on guide cette membrane flottante (2) en fixant sa périphérie à la paroi de ladite enceinte (1), on fixe une bobine motrice (14) au centre de ladite membrane flottante (2), on place cette bobine motrice (14) dans un entrefer (E) que l'on oriente dans le sens de déformation de ladite membrane flottante (2), on mesure le débit instantané d'air sortant par ladite évacuation (6, 8) et on alimente ladite bobine motrice (14) en calculant en continu l'intensité instantanée et le sens du courant d'alimentation en fonction de la pression de consigne de l'air insufflé, dudit débit instantané et des constantes dudit appareil.

2. Procédé selon la revendication 1, **caractérisé en ce que** l'on mesure ledit débit instantané en détectant en continu la variation de position axiale de ladite membrane flottante (2) en fonction du temps.

3. Appareil d'assistance respiratoire, **caractérisé en ce qu'**il comporte une enceinte de compression (1), une membrane flottante élastique (2), divisant cette enceinte de compression (1) en deux compartiments (3, 4) reliés chacun à une admission (5, 7) d'air ou de gaz respiratoire et à une évacuation (6, 8) de gaz comprimé, guidée par sa périphérie fixée à la paroi de ladite enceinte (1), une bobine motrice (14) fixée au centre de ladite membrane (2), un entrefer (E) dans lequel ladite bobine motrice (14) est positionnée, cet entrefer (E) étant orienté dans le sens de déformation de ladite membrane flottante (2), des moyens (24, 25) pour mesurer le débit de ladite enceinte de compression (1) et un processeur de traitement de signal numérique (DSP) pour le calcul de l'intensité instantanée du courant d'alimentation de ladite bobine motrice (14).

4. Appareil selon la revendication 3, **caractérisé en ce que** ladite membrane (2) est associée à au moins deux éléments de guidage élastiques (17, 18) électriquement conducteurs pour connecter ladite bobine motrice (14) à une source d'alimentation commandée par ledit module de traitement de signal numérique (DSP).

5. Appareil selon la revendication 4, **caractérisé en ce que** lesdits moyens de guidage et de connexion comportent au moins deux bras (17, 18), en alliage Cu-Be, disposés symétriquement par rapport au centre de ladite membrane (2).

6. Dispositif selon l'une des revendications 3 à 5, **caractérisé en ce que** ladite bobine motrice (14) et ledit entrefer (E) forment un moteur électrodynamique.

7. Dispositif selon l'une des revendications 3 à 5, **caractérisé en ce que** ladite membrane flottante élastique (2) est fixée à la paroi de ladite enceinte (1) avec une pré-tension diamétrale de 3 à 8% d'allongement.

Fig. 1

Fig. 2

Fig. 3

**Office européen des brevets**

## RAPPORT DE RECHERCHE EUROPEENNE

Numéro de la demande

EP 00 81 1237

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y,D | FR 2 733 688 A (SAIME SARL) 8 novembre 1996 (1996-11-08) * page 5, ligne 8 - page 11, ligne 30; figures 1-3 * | 1,3 | A61M16/00 |
| Y | EP 0 217 573 A (GOULD INC) 8 avril 1987 (1987-04-08) * page 35, ligne 4 - page 37, ligne 4; figures 6,7,9 * | 1,3 | |
| A | FR 1 525 881 A (CESAREO CUBILLAS BARRIO) 9 septembre 1968 (1968-09-09) * figure 1 * | 1,3 | |
| A | WO 95 31241 A (ENGSTROM MEDICAL AB ;BROEMSTER LEIF (SE)) 23 novembre 1995 (1995-11-23) | | |

**DOMAINES TECHNIQUES RECHERCHES (Int.Cl.7)**

A61M

Le présent rapport a été établi pour toutes les revendications

| Lieu de la recherche | Date d'achèvement de la recherche | Examinateur |
|---|---|---|
| LA HAYE | 15 mai 2001 | Zeinstra, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 00 81 1237

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-05-2001

| Document brevet cité au rapport de recherche | | Date de publication | Membre(s) de la famille de brevet(s) | | Date de publication |
|---|---|---|---|---|---|
| FR 2733688 | A | 08-11-1996 | AUCUN | | |
| EP 0217573 | A | 08-04-1987 | US | 4719910 A | 19-01-1988 |
| | | | AT | 74283 T | 15-04-1992 |
| | | | AU | 6234686 A | 19-03-1987 |
| | | | CA | 1284603 A | 04-06-1991 |
| | | | DE | 3684653 A | 07-05-1992 |
| | | | JP | 62194867 A | 27-08-1987 |
| | | | US | 4747402 A | 31-05-1988 |
| | | | US | 4805612 A | 21-02-1989 |
| FR 1525881 | A | 09-09-1968 | AUCUN | | |
| WO 9531241 | A | 23-11-1995 | SE | 504052 C | 28-10-1996 |
| | | | AU | 2542095 A | 05-12-1995 |
| | | | EP | 0758912 A | 26-02-1997 |
| | | | JP | 10500198 T | 06-01-1998 |
| | | | SE | 9401661 A | 14-11-1995 |

EPO FORM P0460